# EUROPÄISCHE PATENTANMELDUNG

(11) **EP 0 995 446 A2**
(43) Veröffentlichungstag der Anmeldung: **26.04.2000**
(21) Anmeldenummer: 99115942.7
(22) Anmeldetag: 13.08.1999
(51) Int. Cl.: A61K 38/57

(54) **Antithrombin III-beta enthaltende Arzneimittelzubereitung**

(30) Priorität: 28.08.1998 DE 19839337
(71) Anmelder: Centeon Pharma GmbH, 35002 Marburg (DE)
(72) Erfinder: Römisch, Jürgen, Dr., 35041 Marburg (DE); Feussner, Annette, 35043 Marburg (DE); Stauss, Harald, 35232 Dautphetal (DE)

(57) **Zusammenfassung**

Es werden Arnzeimittelzubereitungen zur parenteralen Anwendung von Antithrombin III beschrieben, die entweder nur die beta-Isoform des Antithrombins III oder eine Mischung der alpha- und der beta-Isoform des Antithrombins III enthalten. Sie können zur Behandlung von Krankheitsbildern verwendet werden, die durch einen Mangel an der beta-Isoform des Antithrombins III, vorzugsweise auch zur therapeutischen oder prophylaktischen Behandlung von thrombotischen Komplikationen oder Erkrankungen eingesetzt, die mit Entzündungsreaktionen verbunden sind.

## Beschreibung

Gegenstand der Erfindung ist eine Arzneimittelzubereitung zur parenteralen Anwendung von Antithrombin III-beta.

Antithrombin III (AT III) ist einer der wichtigsten plasmatischen Inhibitoren. AT III gehört zu der Familie der Serinprotease-Inhibitoren, die mit ihren "Zielproteasen" einen einer kovalenten Bindung nahekommenden Komplex bilden. Dieser Komplex ist unter physiologischen Bedingungen sehr stabil und wird in der Regel schnell aus dem Blutkreislauf eliminiert. Die Reaktion zwischen AT III und Protease wird durch Heparin drastisch beschleunigt, wobei das AT III nach Assoziation mit dem zur Gruppe der Glykosaminoglykane gehörenden Heparin eine leichte Konformationsänderung erfährt und damit eine beschleunigte Reaktion mit der Protease eingehen kann. Diese Vorgänge spielen physiologisch besonders an Zelloberflächen eine Rolle, welche Glykosaminoglykane z.B. vom Typ der Heparansulfates enthalten und dadurch die Zellen und das Gewebe vor überhöhter proteolytischer Aktivität schützen.

Daneben kommt aber auch der plasmatischen Gerinnung und deren Regulation eine wichtige Bedeutung zu. Im gesunden Zustand zirkulieren keine oder unbedeutende Mengen an Glykosaminoglykanen, so dass diese Vorgänge vor allem durch die progressiven, also Heparin unabhängigen und damit weitaus langsameren Inhibitonscharakteristiken des Antithrombins III bestimmt werden.

Dies ist sinnvoll, um einen Wundverschluß zu gewährleisten, jedoch eine nachfolgende überschießende Gerinnungsaktivierung zu vermeiden.

Besonders deutlich wird die regulatorische Funktion des AT III, wenn sein Plasmaspiegel sinkt, was im Verlauf vieler Krankheiten und besonders drastisch z.B. im Fall einer disseminierten intravasalen Gerinnung (DIC) beobachtet werden kann. Bereits ein Unterschreiten von 70% der entsprechenden Plasmakonzentration ist mit einer drastischen Erhöhung der Mortalitätswahrscheinlichkeit verbunden. Ein Überwiegen von Gerinnungsprozessen führt häufig zu thrombotischen Verschlüssen von Gefäßen und damit zum Organversagen. Deshalb hat sich die Applikation von AT III-Konzentraten aus humanem Plasma besonders in Fällen von angeborenen oder erworbenen Mangelzuständen als sehr hilfreich erwiesen.

Um den beschriebenen pathophysiologischen Vorgängen entgegenzuwirken, wird - neben der vorstehend genannten Substitution mit AT III-Konzentraten - manchmal auch Heparin verabreicht, um das Übergewicht des proteolytischen Potentials auf ein normales Maß zu reduzieren. Dies kann jedoch nur dann funktionieren, wenn genügend AT III vorhanden ist. Da die Verabreichung des Heparins allerdings auch mit einem erhöhten Blutungsrisiko vergesellschaftet ist, das umso höher ist, je dysregulierter die Blutgerinnung ist, muß ständig zwischen pro- und antikoagulatorischen Vorgängen abgewogen werden.

Besonders die Inhibition der Proteasen der Blutgerinnung, wie dem Thrombin sowie den aktivierten Faktoren X und IX, durch AT III ist hier bedeutsam. Eine Verstärkung der progressiven Hemmeigenschaften, die zur Regulation der Hämostase beiträgt, jedoch die Blutungsneigung nicht wesentlich vergrößert, wäre als ein erheblicher Fortschritt anzusehen. Dies gilt auch für inflammatorische Reaktionen, wobei sowohl Thrombin als auch FXa als Bindeglieder zwischen Gerinnung und Entzündungsreaktion angesehen werden können. Eine moderate Inhibition dieser Faktoren hätte also einen Einfluss auf beide Systeme.

AT III zirkuliert im Plasma in einer Konzentration von ca. 150 mg/l. Etwa 90 - 95% des AT III sind an vier Stellen des Moleküls glykosyliert. Diese Isoform wird AT III-alpha genannt. Dagegen besitzt die beta-Isoform nur drei Glykosylierungsstellen. Ein Vergleich dieser beiden Isoformen hat gezeigt, daß eine der Kohlenhydratketten des AT III-alpha in der Nähe der Heparin-bindenden Domäne des AT III lokalisiert ist, jedoch in der beta-Isoform fehlt. Daraus resultiert letztlich eine erhöhte Affinität der beta-Isoform zu Heparin. Studien am subendothelialen Gewebe haben gezeigt, dass dort bevorzugt AT III-beta gebunden ist, vermutlich an den exponierten Glykosaminoglykanen. Bisher ist jedoch nicht bekannt, ob die unterschiedliche Glykosylierung einen Einfluss auf die Progressivhemmung plasmatischer Prozesse hat.

Überraschenderweise wurde nun gefunden, dass die beta-Isoform von Antithrombin III die proteolytischen Aktivitäten der aktivierten Gerinnungsfaktoren F X und F IX in Abwesenheit von Heparin besser inhibiert als die alpha-Isoform.

Gegenstand der Erfindung ist deshalb eine Arzneimittelzubereitung zur parenteralen Anwendung von Antithrombin III, die nur die beta-Isoform von Antithrombin enthält.

Ein weiterer Gegenstand der Erfindung ist eine Arzneimittelzubereitung, die eine Mischung der alpha- und der beta-Isoform von Antithrombin III enthält, in der die beta-Isoform mindestens 15 Gew.-% der Gesamtmenge des Antithrombins ausmacht. Die Herstellung der beiden Isoformen des Antithrombins III und ihre Inhibitionseigenschaften sind im Beispiel 1 näher erläutert.

Die signifikante, jedoch moderat erhöhte Hemmkapazität der beta-Isoform kann aus den vorstehend genannten Gründen bei Patienten mit angeborenen oder erworbenen AT III-Betamangelzuständen durch den Einsatz einer parenteral anzuwendenden Arzneimittelzubereitung behoben werden, die nur die beta-Isoform von Antithrombin oder die beta-Isoform in einem Anteil von mindestens 15 Gew.-% der Gesamtmenge des Antithrombins enthält. Die Applikation dieser Mischung beider Isoformen ist bei AT III beta-Mangelzuständen sinnvoll, weil diese Arzneimittelzubereitung einen höheren Gehalt an der beta-Isoform des Antithrombins aufweist als natürliche AT III-Konzentrate.

Gegenstand der Erfindung ist auch eine Arzneimittelzubereitung, die neben der reinen beta-Isoform des Antithrombins III oder einem Antithrombin III mit einem Gehalt von mindestens 15 Gew.-% der beta-Isoform des Antithrombins noch ein Glykosaminoglykan, insbesondere Heparin enthält. Mit einem derartigen Präparat werden die inhibitorischen Reaktionen der beta-Isoform des Antithrombins erheblich beschleunigt.

Die beta-Isoform kann in reiner Form oder als Bestandteil eines AT III-Konzentrats auch zur Prophylaxe oder Therapie von Krankheiten verwendet werden, die möglicherweise keine deutliche Verminderung der AT III-Plasmakonzentration aufweisen. Das kann z.B. der Fall sein bei Beinvenenthrombosen, bei der Herzinfarktprophylaxe, bei der Rethrombosierungsprophylaxe oder bei septischen Erkrankungen wie der Sepsis oder dem septischen Schock. Die gesteigerte Hemmwirkung gegenüber den aktivierten Blutgerinnungsfaktoren X oder IX lassen den Einsatz der reinen beta-Isoform des Antithrombins als auch eines an beta-Isoform angereicherten Antithrombinkonzentrats als sinnvoll erscheinen. Die Verabreichung einer an der beta-Isoform des Antithrombins- reichen Arzneimittelzubereitung ist praktisch mit keinem erhöhten Risiko einer Blutungsneigung verbunden. Die effektivere Inhibition der aktivierten Blutgerinnungsfaktoren IX und X ist mit einem antiinflammatorischen Effekt verbunden, der deshalb auch zur Prophylaxe und Therapie dieser Zustände ausgenutzt werden kann.

Die erfindungsgemäße Arzneimittelzubereitung kann zur Injektion oder Infusion eingesetzt werden. Sie wird im allgemeinen als Trockensubstanz in einer Durchstechflasche zusammen mit einem getrennt verpackten Lösungsmittel in den Handel gebracht. Die in einer Durchstechflasche, zur einmaligen Verabreichung vorgesehenen Menge des Wirkstoffes beträgt in der Regel zwischen 250 und 2500 I.E. Als Lösungsmittel wird je nach Formulierung der lyophilisierten Lösung Wasser (pro injectione), eine Ringer-Lactat-Lösung oder eine isotonische wässrige Lösung verwendet, die Natriumzitrat, Glukose, Natriummonohydrogenphosphat, eine oder mehrere Aminosäuren aber auch Humanalbumin (aus Plasma, rekombinant oder transgen hergestellt) oder eine Plasmaersatz-Infusionslösung wie Haemacel® enthalten kann. Auch in Form eines parenteral anzuwendenden Flüssigpräparates kann Antithrombin III-beta angeboten werden. Bei gleichzeitiger Gabe von Heparin oder einem anderen Glykosaminoglykan soll dessen Dosierung 500 I.E./Std. nicht überschreiten.

Die Erfindung wird durch die folgenden Beispiele näher erläutert.

### Beispiel 1:

Antithrombin III-alpha und -beta wurden durch Adsorption des AT III-Konzentrates Kybernin®P (Centeon Pharma GmbH, Marburg) an Heparin-Fractogel® und stufenweise Elution gewonnen. Dabei wurde die unterschiedliche Affinität der Isoformen zu Heparin zur Trennung genutzt, wobei AT III-alpha bei niedrigerer und AT III-beta entsprechend bei höherer Salzkonzentration eluiert wurden. Die Isoformen wurden u.a. durch ihr unterschiedliches Laufverhalten bzw. Bandenmuster durch SDS-PAGE und isoelektrische Fokussierung charakterisiert. Die Präparationen zeichneten sich durch sehr hohe Reinheit aus. Die Proteinkonzentrationen wurden nach der Methode von Kjeldahl bestimmt.

Zur Abschätzung der inhibitorischen Potenzen beider Isoformen wurden aus Plasma isolierte, aktivierte Faktoren mit steigenden Konzentrationen der Inhibitor-Isoformen (basierend auf Gesamtproteingehalt nach Kjeldahl) in Abwesenheit oder Anwesenheit von Heparin (2 IU/150µg ATIII) versetzt und die verbleibenden amidolytischen Aktivitäten der Protease mit Hilfe chromogener Peptidsubstrate photometrisch bestimmt.

Dazu wurden die aktivierten Formen der Faktoren II (Thrombin), des F IX, des F X und des Plasminogens (Plasmin) verwendet. Die chromogenen Substrate (3 mM) wurden von der Firma Chromogenix AB (Schweden) gekauft.

**Tabelle 1**

| **Protease** | **Quelle** | **Konz. im Test (IU/ML)** | **Chromogenes Substrat** |
|---|---|---|---|
| Human-α-Thrombin | Centeon | 0.5 IU/ml | S 2238 |
| FIXa | ERL (UK) | 2 µg/ml | S 2288 |
| FXa | Stago/Boehringer (Deutschland) | 0.05 IU/ml | S 2765 |
| Plasmin | Centeon | 0.1 CTA/ml | S 2251 |

| | | | |
|---|---|---|---|
| S 2238 : H-D-Phe-Pip-Arg-pNA x 2HCl | | | |
| S 2288 : H-D-Ile-Pro-Arg-pNA x 2HCl | | | |
| S 2765 : Z-D-Arg-Gly-Arg-pNA x 2HCl | | | |
| S 2251 : H-D-Val-Leu-Lys-PNA x 2HCl | | | |

### Teil A

### Testansatz:

50 µl ATIII-alpha oder-beta
+ 100 µl Puffer
+ 50 µl Protease (siehe Tabelle 1)
+ 50 µl chromogenes Substrat

Bei den Ansätzen mit Heparin wurde die Mischung vor Zugabe des chromogenen Substrates für 10 min bei Raumtemperatur inkubiert. Die Änderung der Absorption bei 405 nm mit der Zeit wurde registriert. Die Inhibition (%) wurde durch Vergleich zum nicht inhibierten Ansatz quantifiziert. Die Konzentrationen von AT III-alpha und AT III-beta, die 50% der Proteasenaktivität inhibierten, wurden ermittelt und als IC50-Wert ausgedrückt (Tabelle 2).

**Tabelle 2**

| **IC 50 (µg/ml)** | | | | |
|---|---|---|---|---|
| | AT III-α | | AT III-β | |
| | -heparin / +heparin | | -heparin / +heparin | |
| Thrombin | 220 | 1.5 | 200 | 0.8 |
| F Xa | 500 | 5.0 | 240 | 3.0 |
| F IXa | 490 | 10.0 | 175 | 7.0 |
| Plasmin | 900 | 55.0 | 900 | 45.0 |

### Ergebnis:

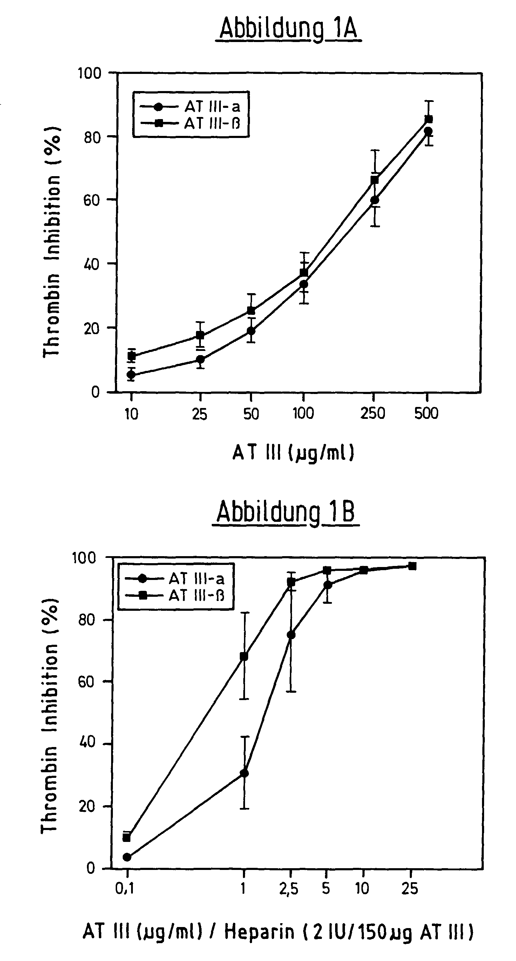

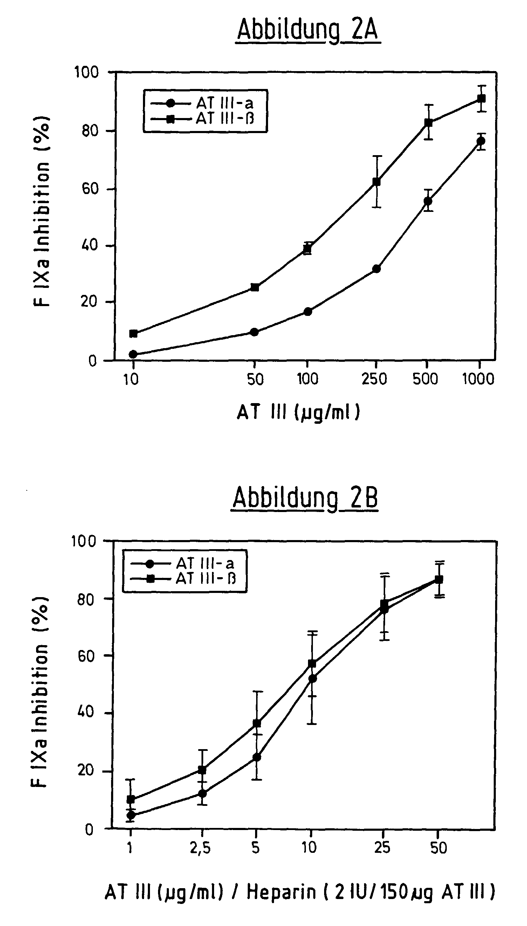

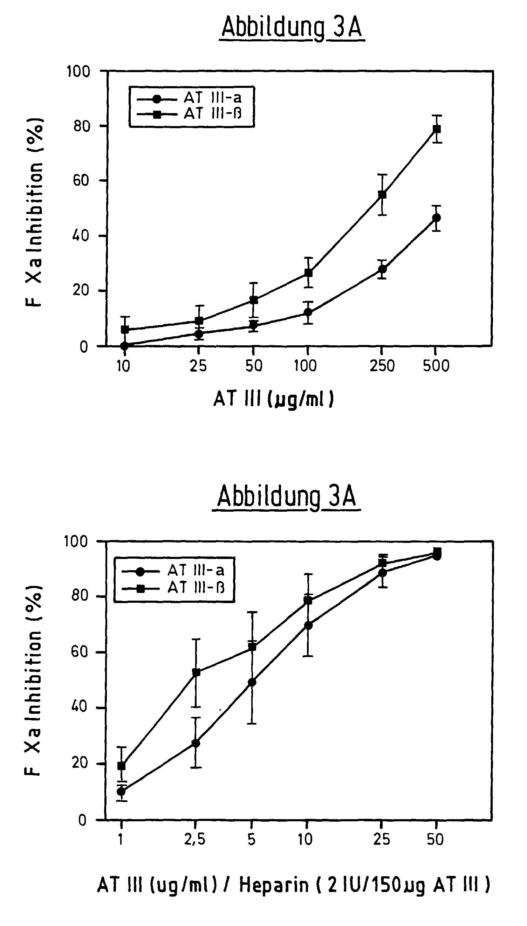

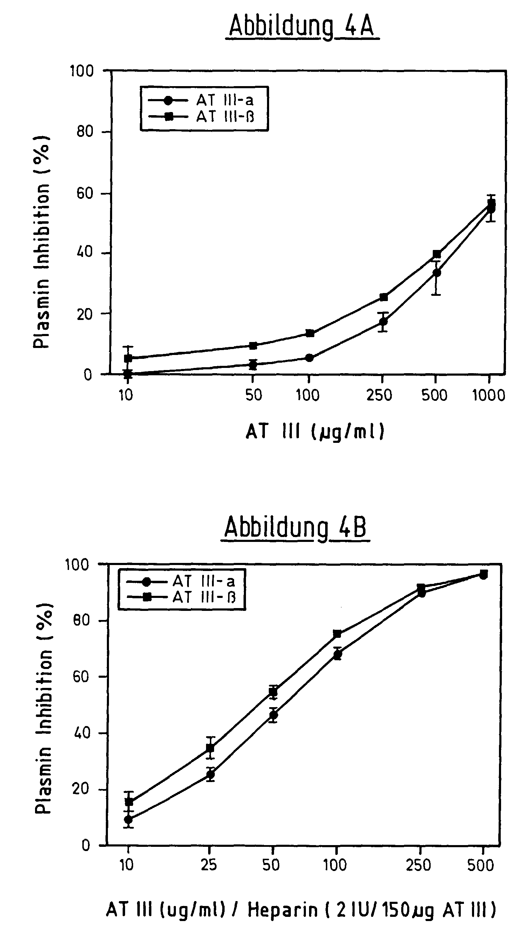

Erwartungsgemäß wurden alle inhibitorischen Reaktionen in Gegenwart von Heparin stark beschleunigt und führten zu einer erheblichen Verringerung der IC50-Werte. Deutliche Unterschiede zwischen beiden Isoformen wurden bedingt durch die Schwankungsbreiten der Tests nicht offenbar (Abbildungen 1-4, jeweils B), jedoch ist gegenüber Thrombin, F IXa und F Xa eine Tendenz zu höherer inhibitorischer Potenz der beta-Isoform zu beobachten.

Im Vergleich zu der alpha-Isoform waren in Abwesenheit von Heparin etwa um die Faktoren 2 und 3 geringere AT III-beta Konzentrationen zur 50%igen Inhibition von F Xa und F IXa notwendig (Abbildungen 1-4, jeweils A).

### Teil B

Die stärkere inhibitorische Potenz des AT III-beta wurde anhand folgenden Experiments weiter vertieft, das entsprechend Beispiel 1A modifiziert durchgeführt wurde:
Während die gesamte Konzentration des im Testansatz eingesetzten AT III's konstant gehalten wurde (bei 100/250/500 µg/ml), wurde der Anteil an AT III-beta erhöht.

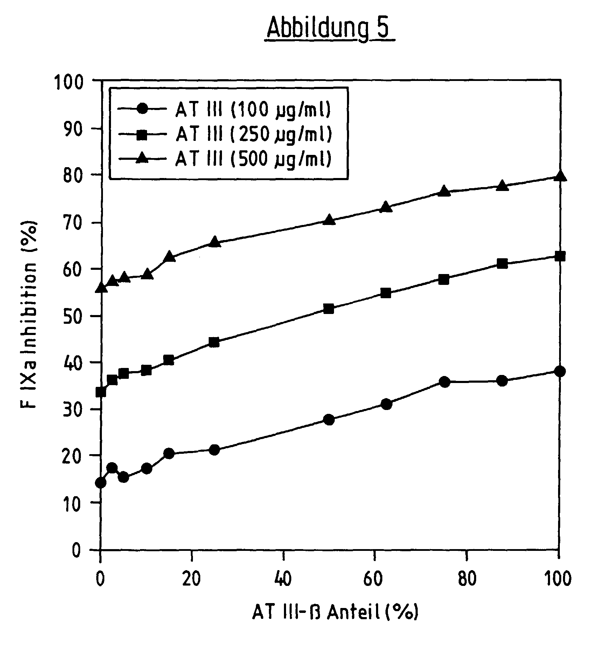

Abbildung 5 zeigt eine mit dem AT III-beta Anteil steigende Inhibition der F IXa-Aktivität. Die Kurven der absoluten AT III-Vorgaben verlaufen entsprechend parallel.

### Beispiel 2

Der Einfluß beider AT III-Isoformen auf die Plasma-Rekalzifizierungszeit wurden mit Hilfe von Koagulometern nach Schnitger und Gross bestimmt. Dazu wurde AT III-Mangelplasma, das keinerlei Antithrombin III enthielt, mit steigenden Mengen an AT III-alpha oder AT III-beta und mit 100 µl Imidazolpuffer versetzt, für 1 Minute bei 37° C inkubiert und danach die Reaktion durch Zugabe von 100 µl Kalziumchlorid (25 mM) gestartet.

### Ergebnis:

Beide AT III-Isoformen führten mit steigender Konzentration zu verlängerten Rekalzifizierungszeiten, wobei AT III-beta die deutlichere Verlängerung bewirkte (Tabelle 3).

**Tabelle 3**

| **Rekalzifizierungszeit (sec)** | | | | |
|---|---|---|---|---|
| AT III-Konzentration (IU/ml) | **AT III-alpha** | | **AT III-beta** | |
| | Mittelwert (n=3) | Standardabweichung (sec) | Mittelwert (n=3) | Standardabweichung (sec) |
| 0 | 217.8 | 3.3 | 217.8 | 3.3 |
| 0.05 | 214.0 | 1.0 | 221.8 | 1.3 |
| 0.1 | 217.0 | 0.6 | 225.0 | 1.0 |
| 0.2 | 220.0 | 1.8 | 225.0 | 3.0 |
| 0.5 | 223.0 | 1.8 | 228.7 | 0.8 |
| 1.0 | 229.3 | 1.8 | 246.0 | 1.5 |
| 2.0 | 241.2 | 0.7 | 258.7 | 0.7 |

## Patentansprüche

1. Arzneimittelzubereitung zur parenteralen Anwendung von Antithrombin III, dadurch gekennzeichnet, daß sie nur die beta-Isoform von Antithrombin III enthält.

2. Arzneimittelzubereitung nach Anspruch 1, dadurch gekennzeichnet, daß sie eine Mischung der alpha- und der beta-Isoform von Antithrombin III enthält, in der die beta-Isoform mindestens 15 Gew.-% der Gesamtmenge des Antithrombins ausmacht.

3. Arzneimittelzubereitung nach den Ansprüchen 1 oder 2, dadurch gekennzeichnet, daß sie als weiteren Wirkstoff ein Glykosaminoglykan enthält.

4. Arzneimittelzubereitung nach Anspruch 3, dadurch gekennzeichnet, daß sie als Glykosaminoglykan Heparin enthält.

5. Verwendung einer Arzneimittelzubereitung nach den Ansprüchen 1 bis 4 zur Behandlung von Krankheitsbildern, die durch einen Mangel an der beta-Isoform des Antithrombins III hervorgerufen werden.

6. Verwendung einer Arzneizubereitung nach den Ansprüchen 1 bis 4, dadurch gekennzeichnet, daß sie zur therapeutischen oder prophylaktischen Behandlung von thrombotischen Komplikationen, septischen Erkrankungen oder Erkrankungen, die mit Entzündungsreaktionen verbunden sind, eingesetzt wird.
